(19) Europäisches Patentamt / European Patent Office / Office européen des brevets

(11) **EP 3 733 959 B1**

(12) **EUROPEAN PATENT SPECIFICATION**

(45) Date of publication and mention
of the grant of the patent:
**01.07.2026 Bulletin 2026/27**

(21) Application number: **20171483.9**

(22) Date of filing: **27.04.2020**

(51) International Patent Classification (IPC):
**D21C 3/26** *(2006.01)*    **D21C 7/12** *(2006.01)*
**D21C 9/00** *(2006.01)*    **D21D 1/00** *(2006.01)*
**D21G 9/00** *(2006.01)*

(52) Cooperative Patent Classification (CPC):
**D21C 9/007; D21C 3/26; D21C 7/12; D21D 1/002; D21G 9/0018**

(54) **MONITORING AND CONTROLLING OF REFINING OF FIBROUS PULP**

ÜBERWACHUNG UND STEUERUNG DER RAFFINIERUNG VON FASERZELLSTOFF

SURVEILLANCE ET COMMANDE DE RAFFINAGE DE PÂTE FIBREUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.05.2019 FI 20195363**

(43) Date of publication of application:
**04.11.2020 Bulletin 2020/45**

(73) Proprietor: **Valmet Technologies, Inc.**
**02150 Espoo (FI)**

(72) Inventor: **KAUPPINEN, Lasse**
**02150 Espoo (FI)**

(74) Representative: **Laine IP Oy**
**Porkkalankatu 24**
**00180 Helsinki (FI)**

(56) References cited:
EP-A1- 2 014 828          EP-A1- 2 740 839
WO-A1-2011/064441    WO-A1-2015/025084
WO-A1-2016/001480    WO-A1-94/08223
WO-A1-99/15877          JP-A- 2013 186 034
US-A- 4 837 446          US-A- 5 786 894

**Description**

FIELD

Various example embodiments relate to monitoring and controlling of refining of a fibrous pulp.

BACKGROUND

Refining of a pulp is one of the most important stages in the paper, board or tissue making process. Refining has effect on operation and energy consumption of the paper, board or tissue machine and chemical dosing strategy. It also determines the quality and final properties of an end product, such as strength and printability. Thus, numerous tests such as fibre length, diameter, modulus and strength, freeness and chemical purity measurements, can be conducted on the pulp fibres to predict and modify properties of the end product.

JP2013186034 A discloses a method for measuring the amount of non-fibrillated cellulose fibres. The method includes steps of observing a sample including cellulose fibres by a polarization microscope, binarizing a polarization microscope observation image obtained by the observation, and calculating the amount of non-fibrillated cellulose fibres per unit area as area-% on the basis of the binarized image.

WO 99 15877 discloses a method for measuring fibre-like particles in a suspension by using an optically measuring instrument.

However, there is still need to improve determining of fibre properties and controlling of fibre modification and chemical dosing for higher quality paper, board, tissue, and microfibrillated, nanofibrillated and nanocrystalline cellulose.

SUMMARY OF THE INVENTION

The method of the present invention is defined in the current claim 1. Furthermore, the present claim 7 defines a apparatus for monitoring and controlling of refining of fibrous pulp. Even further, the current claim 15 refers to a fibrous pulp processing system according to any of the preceding claims 7 to 14.

The pulp sample is a pulp liquid suspension.

According to an embodiment, the controlling fibre refining comprises controlling of further processing of pulp batch being analysed and refined into a further or a recurrent refining stage or to a subsequent process stage after the pulp refining.

According to an embodiment, the apparatus is provided before a headbox in a paper, board or tissue machine.

According to an embodiment, pulp refining is controlled for microfibrillated, nanofibrillated and nanocrystalline cellulose manufacturing process.

According to an embodiment, internal fibrillation is analysed on the basis of the relation between fibrillated fibres and all fibres or non-fibrillated fibres in the pulp and/or by energy used in the refining of the pulp.

According to an embodiment, the means are configured to generate the control parameter further on the basis of Canadian standard freeness parameter and/or Schopper Riegler parameter.

BRIEF DESCRIPTION OF THE DRAWINGS

FIGURE 1 illustrates a pulp manufacturing process in accordance with at least some embodiments of the present invention,

FIGURE 2 illustrates a method for monitoring and controlling of refining of the fibrous pulp in accordance with at least some embodiments of the present invention,

FIGURE 3 illustrates an apparatus for monitoring and controlling of refining of the fibrous pulp in accordance with at least some embodiments of the present invention,

FIGURE 4 illustrates fibres in a pulp sample before refining,

FIGURE 5 illustrates fibres in a pulp sample after refining, and

FIGURE 6 illustrates fibres in a pulp sample after second stage of refining.

EMBODIMENTS

In the present context, the term "pulp" includes but is not limited to pulps made from softwood, hardwood or non-wood fibres, and recycled pulps made from printed waste papers such as newspapers, advertising leaflets, magazines, data recording papers, photocopies, computer print outs or mixtures of these printed matters such as waste magazine papers and office waste papers as well as mixtures thereof. The term also comprises microfibrillated (MFC), nanofibrillated (NFC) and nanocrystalline (NCC) cellulose, synthetic pulp and artificial pulp.

In the present context, the term "fibrillation" comprises the shearing of pulp fibres to loosen fibrils from the fibre surface and fibre wall by external fibrillation and/or delamination and swelling of fibres by internal fibrillation.

In the present context, the term "fibrillated fibre" comprises fibres that have been processed to develop fibres with a higher surface area and/or branched structure.

In the present context, the term "refining" comprises mechanical and/or chemical treatment for fibrillation of the fibrous pulp.

In the present context, the term "chemical fibrillation" comprises chemical treatment for fibrillation of the fibrous pulp.

In the present context, the term "approach flow system" comprises the part of the papermaking process between the final stock preparation storage tanks and the headbox of the paper machine.

During refining of a pulp, fibrillation of cellulose fibres takes place. Fibrillation is one of the most important fibre properties for controlling properties of an end product. For example, it has been noted that fibrillated fibres make more strength to the end product by stronger fibre to fibre bonding by the increased bond area of the fibres. In addition, studies with functional paper chemicals have shown that chemicals tend to attach more fines and fillers for more fibrillated fibres and microfibrillated, nanofibrillated and nanocrystalline cellulose. Thus, it is important to measure fibre properties, particularly fibrillation, for controlling refining, optimizing chemical dosing strategy, and modelling strength and other mechanical properties of the end product. However, there is still need for improve determining and controlling of the fibrillation. Thus, the present embodiments provide an improved method for monitoring and controlling refining of a fibrous pulp.

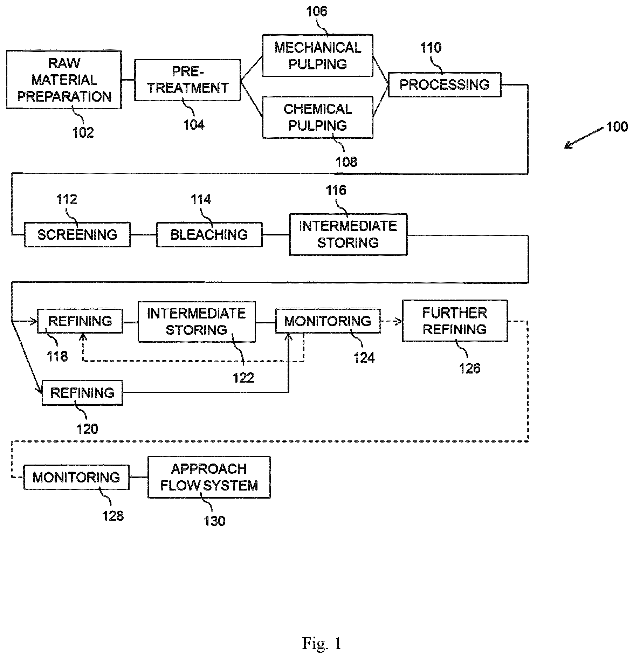

FIGURE 1 illustrates a pulp manufacturing process 100. The process may comprise raw material preparation 102, pre-treatment 104, mechanical or chemical pulping 106, 108, processing 110, screening 112 and bleaching 114 of the pulp, intermediate storing 116, 122 of the pulp, refining 118, 120 of the pulp, monitoring 124 of the pulp, further refining 126 of the pulp.

The pulp may be again monitored 128 after the further refining 126. The pulp may be provided for further process stages. In the example of FIGURE 1, the pulp is provided to an approach flow system (AFS) 130. Approach flow system steps 130 may comprise for example, a headbox of a paper, board or tissue machine, a mixing tank, a machine tank, a chemical treatment or a steam explosion system. It should be appreciated that FIGURE 1 illustrates only one example of the pulp manufacturing process and at some of the stages may be different or even omitted. The pulp manufacturing process may also comprise other process stages, such as pre- and post-treatments and intermediate storing of the pulp. Furthermore, refining and monitoring may be carried out at several stages.

The raw material preparation 102 may comprise for example, debarking, chipping, cooking, bleaching, screening and washing of the raw material. In chipping, the logs (or portions of logs) are reduced to chip fragments suitable for the subsequent pulping operations. Screening may segregate chips on the basis of chip length and/or thickness.

Mechanical or chemical pulping 106, 108 is used to disintegrate wood (or other fibrous raw material) individual cellulose fibres. The mechanical pulping methods use mechanical energy to weaken and separate fibres from wood via a grinding action. Mechanical pulping methods comprise for example, groundwood (GW), refiner mechanical (RMP), thermomechanical (TMP) and chemi-thermomechanical (CTM) pulping. Chemical pulping breaks down the bulk structure of the fibre source by degrading the ligning and hemicellulose into small, water-soluble molecules which can be washed away from the cellulose fibres. Chemical pulping methods comprise for example, the kraft process and sulfite process.

After pulp production, pulp processing 110 removes impurities, such as uncooked chips, and recycles any residual cooking liquor via the pulp washing process. Screening 112 of the pulp is done to remove oversized and unwanted particles such as knots and shives. Then, the pulp may be bleached to get lighter color. Bleaching 114 may be also used for purification of the pulp by removing hemicelluloses and wood extractives as well as lignin.

Pulp produced in a pulp mill without any mechanical treatment is usually unsuitable for most paper grades. Thus, refining 118 is conducted for achieving better pulp quality. In the refining process, fibres become fibrillated by external fibrillation as outer portions of the fibres are peeled at least partly. Some of the outer portions of the fibres may stay attached and increase strength of the fibre. This significantly increases the surface area of the fibres. In addition, the inner wall may become delaminated by internal fibrillation which increases the conformability, flexibility, bonding tendency and thickness of the fibres. Due to internal fibrillation, larger fiber-to-fiber bonding surface and more bulk is obtained. In addition, dewatering is more effective due to lower amount of fines and the need for drying energy is reduced.

Refining 118 may be carried out by for example, a beater or a refiner, such as a single disc refiner, a conical disc refiner, a double disc refiner or a flat disc refiner. The refiner may comprise of two blades facing and rotating relative to one another creating the mechanical action of refining.

Referring to FIGURE 1, the refining process may comprise simultaneous refining 120 and/or further refining 126 stages. In the simultaneous refining, the pulp flow may be divided and guided to at least two refiners which refine the fibres at the same time. Refining may comprise for example, 1 to 10 stages, especially 1-5 stages, preferably 1-3 stages. Refining stage(s) can be repeated to generate fibrillated fibres. Refining may be monitored and controlled to achieve preferred fibre properties. The pulp can be guided to a further or back to recurrent refining stage on the basis of a fibre monitoring 124 to achieve preferred fibre properties. For example, after the fibre monitoring 124, the fibres may be guided to previous refining 118 stage or further refining 126 stage. The fibres may be guided through the same refining stage several times, even up to 20-30 times. Then, the fibres may be guided again to the fibre monitoring 124. The fibre monitoring and previous or further refining stages may be repeated until the preferred fibre properties are achieved. When the preferred fibre properties are achieved, the pulp may be provided to a further process steps.

In the following examples, the term fibrillated fibres proportion parameter is used to refer to relation between fibrillated fibres and all fibres or non-fibrillated fibres in the pulp.

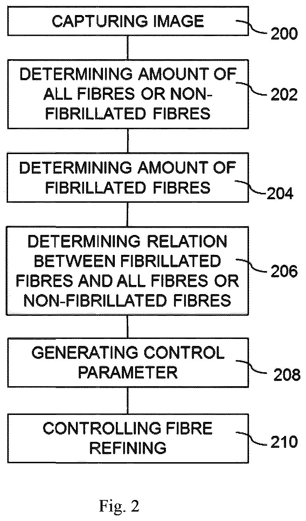

FIGURE 2 illustrates the method for monitoring and controlling of refining of the fibrous pulp. The method may comprise capturing 200 at least one image of a pulp sample, determining 202 amount of all fibres or non-fibrillated fibres in the at least one image, determining 204 amount of fibrillated fibres in the at least one image, determining 206 relation between fibrillated fibres and all fibres or non-fibrillated fibres in the pulp on the basis of the amount of fibrillated fibres and the amount of all fibres or non-fibrillated fibres in the at least one image, generating 208 a control parameter on the basis of the determined relation between fibrillated fibres and all fibres or non-fibrillated fibres in the pulp, and controlling 210 fibre refining by at least one pulp refining device on the basis of the control parameter. Due to monitoring and controlling of refining of the fibrous pulp the smooth operation of the paper, board or tissue machine can be achieved, and final properties of the pulp or the end product can be adjusted. In addition, chemical dosing strategy can be optimized and strength and other mechanical properties of the end product can be modelled according to determined fibrillated fibres proportion parameter.

In addition to the determined relation between fibrillated fibres and all fibres or non-fibrillated fibres in the pulp, the means may be configured to generate 208 the control parameter further on the basis of Canadian standard freeness (CSF) parameter and/or Schopper Riegler (SR) parameter. CSF and SR parameters give a measure of the drainability of a pulp suspension in water. CSF parameter may be measured according to standards TAPPI T227 and ISO 5267-2. SR parameter may be measured according to standard 5267-1. Moreover, other inputs may be used to generate the control parameter 208.

It is noted that since the method of FIGURE 2 may be implemented in an apparatus separate from imaging device, block 200 may refer to receiving image data of the pulp sample.

Controlling 210 fibre refining may comprise for example, adjusting of a blade gap between segments in a refiner. The space between the blades can be widened or shortened, depending on the extent of refining appropriate to the end-use of the pulp, paper, board or tissue to be produced. The blade gap can be measured by at least one sensor or indirectly from refiner vibration or other indicative measurement.

The blade gap between the segments in the refiner has a major impact of both the production rate as well as the pulp quality. In addition, an optimal blade gap lowers refiner lifecycle costs due to fewer production stops and better refiner performance. Therefore, it is of great importance that the refiner runs with the optimal blade gap.

In addition, controlling 210 fibre refining may comprise controlling of further processing of pulp batch being analysed and refined into a further or a recurrent refining stage or to a subsequent process stage after the pulp refining. The refining stages may be repeated until the pulp fulfils set requirements. Fibrillated fibres proportion parameter and other fibre properties can be determined before and/or after every refining stage.

Controlling 210 fibre refining may also comprise controlling of changing of blades of the refiner. The blade may be changed according to indication generated on the basis fibrillated fibres proportion parameter. The blades with different patterns may be used for different stages of refining. The optimal selection of the blades provides preferred fiber properties and may reduce need for several refining stages.

Moreover, controlling 210 fibre refining may comprise controlling of dilution water. An increase of dilution water intake reduces the pulp consistency. When operating at undesirably high dilution water intake, high pulp flow inside of the segments of the refiner may occur. This can lead to low pulp quality due to short refining time inside the segments and low specific energy input. On the contrary, decrease in dilution water intake gives higher consistency. This can lead to a lowering of intensity due to inadequate pulp flow between the segments, causing inadequate pulp quality and plugging of the segments. Thus, controlling of dilution water is important for achieving optimal fiber properties and pulp flow.

Controlling 210 fibre refining may also comprise controlling of production volume. Production volume may be controlled by a feeder screw or a pump. Production volume impact on retention time of the pulp and the pulp and steam flow. Thanks to optimal production volume, the refiner is stabilized due to even inflow of the pulp.

According to some embodiments, controlling 210 fibre refining may comprise controlling chemical treatment for

**EP 3 733 959 B1**

chemical fibrillation of the fibrous pulp on the basis of the control parameter. The controlling chemical treatment may comprise controlling of dosage and/or duration of application of chemicals, controlling enzymatic treatment, controlling of dispersant treatment, controlling of solvent treatment, controlling of chemical additive or agent treatment to promote or enhance hydrolysis of cellulose, controlling of post-refining treatment, controlling retention chemical treatment and/or controlling amount of water. Chemicals may comprise for example, retention chemicals, polyacrylamide (PAM), silica and chemicals used in MFC or NFC production, such as 2,2,6,6-tetramethylpiperidine-1-oxyl (TEMPO). Enzymes may comprise for example, cellulase, xylanase, laccase and lipase.

The controlling of dosage of chemicals may be used as pre-treatment for refining and/or after and/or between the stages of mechanical refining. Chemical may be dispensed directly to a pulp container, during process stages or in the approach flow system. Chemicals may be dispensed after pulping or after a preliminary refining in MFC or NFC process. Dispensing PAM and silica may be carried out for example, in a short circulation of a paper machine. Dispensing of the retention chemicals may be carried out for example, in the approach flow system.

The duration of application and dosage of the chemicals may be optimized to reach preferred ratio of fibrillated fibres. The dosage of chemicals may be controlled to form indurated fibres and for softening of the upper layer of the fibres. Chemicals may increase internal fibrillation of the fibres by delamination, wherein a wall of the fibre thickens when the walls of the fibres are separated. Controlling dosage of chemicals produce fibres with optimal properties such as strength. In addition, enzymatic pre-treatment for refining may improve efficiency of refining process.

Before pulp monitoring 124 and determining of properties of the fibres, a pulp sample is prepared. The sample preparation may be manual or automatic. The pulp sample may be a pulp liquid suspension, such as a pulp water suspension. The pulp sample properties, such as weight and volume of the sample, may be determined before controlling of a consistence of the sample and determination of the fiber properties of the sample. The consistency of the pulp liquid suspension may be controlled for example, by adding sufficient amount of liquid, such as water, to the suspension. A wetting agent may be added to the suspension to improve uniform distribution of the fibres in the suspension. The wetting agent may be for example, a surfactant or an alcohol.

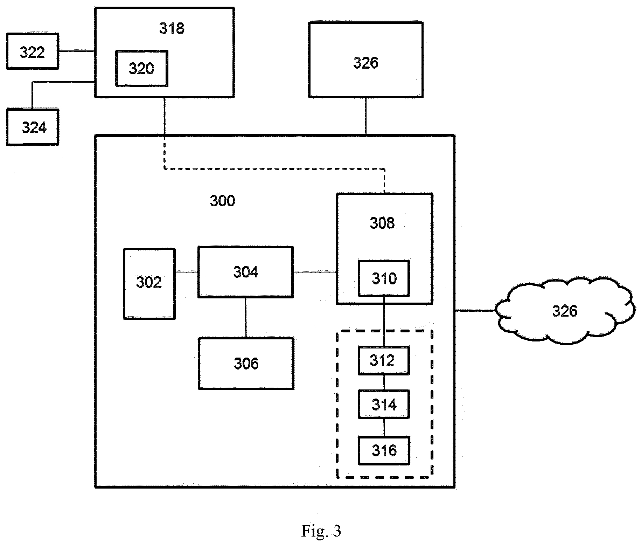

The amount of the fibrillated and/or all fibres may be determined 202, 204 by a fibre classifier, such as the fibre classifier 314 illustrated in the apparatus of FIGURE 3, configured to: detect fibres and fibrils of fibres in the at least one image, and classify the detected fibre as a fibrillated fibre in response to fibrils connected to the fibre reaching a predetermined threshold parameter. The fibre classifier may be a part of the apparatus or connected to the apparatus. The predetermined threshold parameter may be set for example, according to the used pulp type or input material type, such as tree species. The predetermined threshold parameter may be for example one to three fibrils, especially one fibril. Due to the fibre classification, fibrillated fibres proportion parameter in the pulp may be determined.

According to some embodiment, an object recognized in the at least one image may be classified as a fibre or a fibril on the basis of determined length and/or width of the object. The object may be classified as the fibre if the length of the object is for example, in the range of 0.5 to 15 mm, and/or if the width of the object is for example, in the range of 10 to 75 $\mu$m. The object may be classified as the fibril if the width of the object is for example, under 10 m$\mu$. The object may be classified as MFC or NFC fibre if the length of the object is for example, in the range of 100 nm to 100 $\mu$m, and/or if the width of the object is for example, in the range of 10 to 100 nm. The object may be classified as MFC or NFC fibril if the width of the object is for example, under 10 nm, especially in the range of 5 to 6 nm. The predetermined threshold parameters for width and length of the fibres and the fibrils may be set for example, according to the used pulp type or tree species. Classification may be done by determining length and/or width of the object at least one image by the fibre classifier 314. Due to the fibre classification, fibrillated fibres proportion parameter may be determined.

An object recognized in the at least one image may be classified as a fibre or a fibril on the basis of determined area of the object. Classification may be done by detecting the objects and determining the area of the objects at least one image by the fibre classifier 314. The predetermined threshold parameter(s) for area of the fibres and the fibrils may be set for example, according to the used pulp type or tree species.

According to some embodiment, a fibre recognized in the at least one image may be classified into the non-fibrillated fibre or the fibrillated fibre on the basis of a determined area or a determined circle of the fibre. Classification may be done by determining the area or the circle of the fibre at least one image by the fibre classifier 314. Due to the fibre classification, fibrillated fibres proportion parameter may be determined.

After the fibre classification, fibrillated fibres proportion parameter in the at least one image may be determined. Determination may be conducted by calculating ratio of the fibrillated fibres by using following equation:

$$Fibrillated\ fibres\ proportion\ parameter = \frac{Amount\ of\ measured\ fibrillated\ fibres}{Amount\ of\ all\ measured\ fibres\ or\ non\text{-}fibrillated\ fibres} \times 100\ \%$$

The above mentioned new parameter for measuring fibre properties gives a useful tool for controlling refining, modelling paper, board or tissue strength and other mechanical properties and optimizing chemical dosing strategy. Due to the parameter, refining of the pulp may be controlled to adjust the ratio of the fibrillated fibres to achieve preferred pulp and/or

5

end production properties. As compared to controlling a fibrous pulp process on the basis of fibrillation index or degree based on relation between total surface or perimeter of fibres and total surface or perimeter of fibrils, controlling fibre refining on the basis of the present ratio enables more accurate control of the refining and the pulp properties. Generally used fibre fibrillation index or degree takes only into account a fibrillation degree of the pulp fibres. Thus, the fibre fibrillation index does not take into account an amount of the non-fibrillated fibres or all measured fibres and their relation to the amount of the fibrillated fibres. Thanks to present fibrillated fibres proportion parameter effect of the non-fibrillated fibers on the pulp or end product properties can also be taken into account. For example, the fibrillated and non-fibrillated fibers may have different chemical treatment response. Thus, detecting also non-fibrillated fibres and calculating the fibrillated fibres proportion parameter is essential for achieving for example, optimal pulp properties in MFC, NFC and NCC manufacturing, and higher web strength, lower porosity, higher smoothness and higher gloss in paper manufacturing. The present fibrillated fibres proportion parameter is also essential in optimizing of amount of non-fibrillated fibres for achieving sufficient water removal properties.

The method of FIGURE 2 may be provided before, after and/or during the paper, board or tissue making process. The method may be provided for example, after chemical or mechanical pulping 106, 108, and before and/or after refining 118, 120, 126. Thus, the method may give useful information about fibre properties in various stages of the paper, board or tissue making process and the process may be controlled according to the fibre properties to achieve an end product with optimal properties.

The method of FIGURE 2 may be provided before a headbox in a paper, board or tissue machine rather than waiting for time-delayed laboratory tests done after paper, board or tissue manufacturing. Thus, the method may give useful information about fibre properties before web formation and fibre refining may be controlled according to the fibre properties to achieve an end product with preferred properties.

The pulp refining may be controlled for MFC, NFC and NCC manufacturing process. Thus, the method may give useful information about fibre properties during cellulose manufacturing and before formation of a product comprising cellulose. Moreover, MFC, NFC and NCC fibre refining may be controlled according to the fibrillated fibres proportion parameter to achieve MFC, NFC and NCC or a MFC, NFC and NCC product with preferred properties.

The means may be configured to generate 208 the control parameter further on the basis of internal fibrillation. In some embodiments, the internal fibrillation may be analysed on the basis of the relation between fibrillated fibres and all fibres or non-fibrillated fibres in the pulp and/or by energy used in the refining of the pulp. The fibre may be classified as an internally fibrillated fibre if the width of the fibre reaches predetermined threshold value. The amount of the internal fibrillation may increase when energy consumption increases in the refining process. Thus, internal fibrillation may be controlled by the fibrillated fibres proportion parameter and/or the energy consumption to achieve an end product with preferred properties.

FIGURE 3 illustrates an apparatus 300 for monitoring and controlling of refining of fibrous pulp, comprising means configured for at least performing: capturing at least one image of the pulp sample, determining amount of all fibres or non-fibrillated fibres in the at least one image, determining amount of fibrillated fibres in the at least one image, determining relation between fibrillated fibres and all fibres or non-fibrillated fibres in the pulp on the basis of the amount of fibrillated fibres and the amount of all fibres or non-fibrillated fibres in the at least one image, generating a control parameter on the basis of the determined relation between fibrillated fibres and all fibres or non-fibrillated fibres in the pulp, and causing control of fibre modification by at least one pulp refining device on the basis of the control parameter. It is to be appreciated that FIGURE 3 illustrates only one example of an applicable apparatus for monitoring and controlling of refining of fibrous pulp.

The apparatus may comprise or be connected to network(s) 326. Thus, the refining may be monitored and controlled fast and accurately already during the refining.

A pulp sample may be prepared in a sample handling unit 302 of the apparatus 300. The sample handling unit may comprise plurality of automatic samplers for example, 1 to 20 samplers, especially 12 samplers. The pulp sample properties, such as weight, volume and pH of the sample, may be measured and the pulp sample may be prepared for imaging by the sample handling unit. Consistency of the pulp sample may be controlled by adding sufficient amount of liquid, such as water, to the suspension.

The pulp samples may be taken at several points of the pulp stock for example, 1 to 20 points. The measurement cycle time may be for example, 0.5 to 10 minutes, preferably 3 to 6 minutes.

The means for capturing at least one image of the pulp sample comprise an imaging module 304. The imaging module device may include a recording device that can record images in a digital format. The imaging module may capture for example, 30-70 frames/s. Size of the image may be for example, 30*20 mm, 8*6 mm for a high-definition (HD) image, and 16*13 mm for an ultra-high-definition (UHD) image.

The apparatus may comprise also further modules or units, such as a freeness measuring module 306. The main goal of freeness control is to produce a pulp that drains consistently and runs well on the paper, board or tissue machine. Consistency of the sample may be controlled automatically. Freeness of the pulp may be measured according to Canadian standard freeness method (CSF) described in standards TAPPI T227 and ISO 5267-2. In addition, Schopper Riegler (SR) parameter may be measured according to standard 5267-1 in the freeness module.

The images may be processed by image processing software after the image capturing. Image processing may comprise for example, filtering, noise reduction, changing of sharpness, brightness, and/or contrast of the images and/or color adjustment of the images. Image processing aid in detection of the objects and in fiber classification.

The apparatus 300 or the imaging module 304 may also comprise illuminating means to assist in the capture of images of the fibres. The illuminating means may comprise a lighting device such as a light emitting diode (LED) or an organic light emitting diode (OLED) light. The illuminating means may be positioned such that light is transmitted through the sample. On the other hand, the illuminating means may be positioned such that light is reflected from the samples. The illumination means aid the imaging module in image capturing and the fibre classifier 314 in detection of the objects and in fiber classification.

The means for determining amount of all fibres or non-fibrillated fibres in the at least one image and the means for determining amount of fibrillated fibres in the at least one image comprise a fibre analysis module 308. The fibre analysis module may be a part of the apparatus or connected to the apparatus. The fibre analysis module may comprise a fibrillation analyser 310, which may comprise or may be connected to a fibre detector 312, a fibre classifier 314, and a fibrillation relation parameter generator 316.

The sample may be automatically diluted to the set consistency which depends on the type of the pulp. After that, the sample suspension may flow as a continuous flow through the imaging module 304. Images may be taken from the sample flow at regular intervals during predetermined time until image amount or confidence interval calculation is achieved. Various objects may be identified and counted by fibre detector 312 from the captured images, such as fiber dimensions, fine particles, fiber fibrillation, fiber bundles, tube cells, and/or sticks. The fibre classifier 314 may classify fibres by automated computer image analysis. Suitable algorithms stored in the computer may enable the fibre classifier to determine dimensions of the objects detected in at least one image. The image analysis is a quick and reliable method for determining the fibres. The amount of the non-fibrillated fibres and fibrillated fibres may be counted by the fibre classifier 314 of the fibrillation relation parameter generator 316. Finally, the fibrillation relation parameter generator 316 may determine the fibrillated fibres proportion parameter.

The fibre analysis module 308 may be used for determining also other properties of the fibres such as details of fines, kink, curl, size distribution, fractions, and hardwood and softwood ratio. The determination of fibrillated fibres proportion parameter, possibly combined with other fibre properties measured by the apparatus, can be processed in a modeling tool that helps to predict how fibre properties will affect final sheet properties.

The apparatus 300 and/or the fiber analysis module 308 may comprise a processor, a communications unit and a memory. The fiber analysis module may comprise a transmitter and/or receiver, which may be configured to operate in accordance with global system for mobile communication, GSM, wideband code division multiple access, WCDMA, long term evolution, LTE, 5G or other cellular communications systems, wireless local area network, WLAN, and/or Ethernet standards, for example.

The memory may store computer program code and parameters for causing the fiber analysis module 308 to perform at least some of the presently disclosed features, such as determining fibrillated fibres proportion parameter and at least some of the other features of FIGURE 2, when the computer program code is executed by the processor. The memory, processor and computer program code may thus be the means to cause the fiber analysis module to perform at least some of the presently disclosed features related to the new fibrillated fibres proportion parameter.

The means for generating a control parameter on the basis of the determined fibrillated fibres proportion parameter comprise a process controller 318. The process controller may comprise a refining controller 320 for generating the control parameter(s) 208 and/or controlling 210 the refining process by applying at least some of the features illustrated above.

The means for causing control of fibre modification by at least one pulp refining device on the basis of the control parameter comprise or be connected to a mechanical refining controller 322 and/or a chemical treatment controller 324 for implementing at least some of the above illustrated mechanical and/or chemical fibre refining actions. The mechanical refining controller may be a control unit in a mechanical refiner and control for example, adjusting of a blade gap between segments in a refiner, pulp batch into a further or a recurrent refining stage or to a subsequent process stage after the pulp refining, changing of blades of the refiner, dilution water and/or production volume. Chemical treatment controller may control for example, dosage of chemicals.

The apparatus may further comprise one or more user interface (UI) 326 devices, such as a display and input means, such as one or more of a keyboard, a touch screen, a mouse, a gesture input device or other type input/output device. The UI may be configured to display the analyzing results and provide user input for controlling the computing unit. It will be appreciated that various other data related to the monitoring and controlling of refining of fibrous pulp may be displayed and/or controlled via the UI.

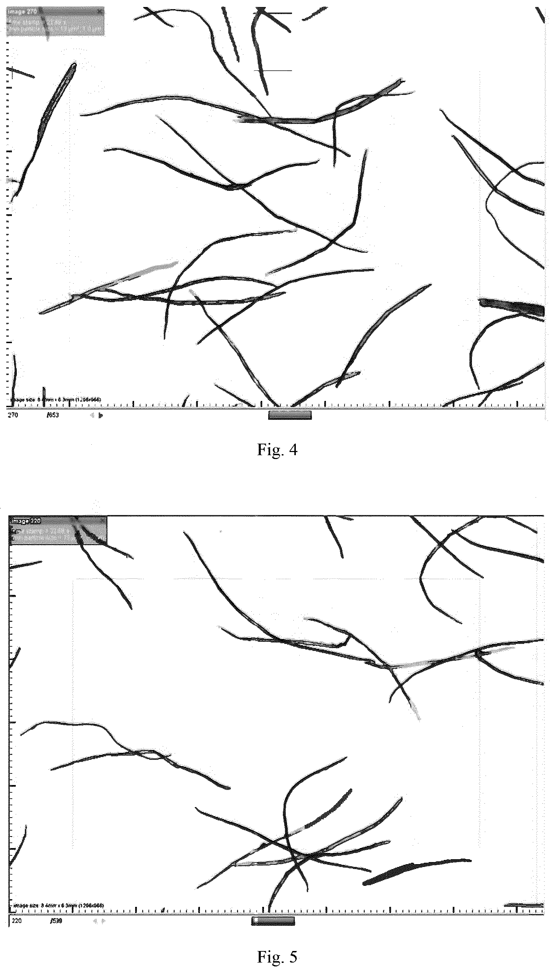

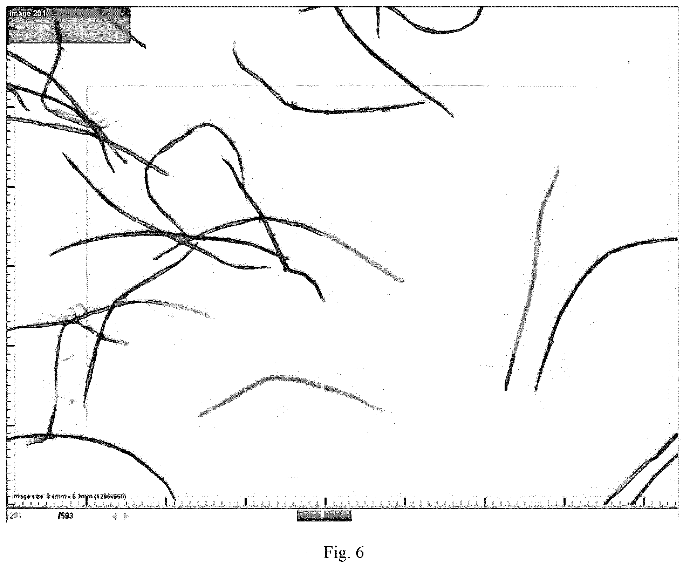

FIGURE 4 illustrates fibres in the pulp sample before refining. Amount of fibrillation is very low. The pulp comprises almost merely plain non-fibrillated fibres. FIGURE 5 shows the refined pulp. Fibrillation is seen due to refining effect, but there are still lot of plain non-fibrillated fibres in the pulp. FIGURE 6 illustrates fibres after the second stage of refining. More fibrillation is observed naturally due to further refining. However, there are also non-fibrillated fibres that have passed the refining stages (nearly) without damage.

The present embodiments have several benefits. Previous methods for monitoring of the pulp were not sufficient for controlling refining. Instead, the present embodiments give the new fibrillated fibres proportion parameter which can be used to effectively control refining and achieve optimal end product properties. Due to controlling of refining by the new parameter, higher web strength can be achieved by higher fibre to fibre bonding. Optimal relation between fibrillated fibres and non-fibrillated fibres may be achieved by the parameter leading to excellent mechanical properties due to right amount of the fibrillated fibres and sufficient water removal properties due to right amount of the non-fibrillated fibres. In addition, better formation of the web, and lower porosity, better printability, higher smoothness and higher gloss of the end product may be achieved. Moreover, chemical dosing strategy and energy consumption can be optimized and strength of the web can be modelled due to controlling of refining by the parameter.

INDUSTRIAL APPLICABILITY

At least some embodiments are feasible in monitoring and controlling of refining of fibrous pulp.

**Claims**

1.  A method for monitoring and controlling of refining of a fibrous pulp, the method comprising:

    - capturing (200) at least one image of a pulp sample by an imaging module (304);
    - determining (202) amount of all fibres or non-fibrillated fibres in the at least one image by a fibre analysis module (308); **characterized by**
    - determining (204) amount of fibrillated fibres in the at least one image based on image processing by the fibre analysis module (308);
    - determining (206) relation between fibrillated fibres and all fibres or non-fibrillated fibres in the pulp on the basis of the amount of fibrillated fibres and the amount of all fibres or non-fibrillated fibres in the at least one image by the fibre analysis module (308);
    - generating (208) a control parameter on the basis of the determined relation between fibrillated fibres and all fibres or non-fibrillated fibres in the pulp by a process controller (318), and
    - controlling (210) fibre refining by at least one pulp refining device on the basis of the control parameter by mechanical refining controller (322).

2.  The method of any preceding claim, wherein the amount of the fibrillated and all fibres is determined by a fibre classifier (314) configured to:

    - detect fibres and fibrils of fibres in the at least one image, and
    - classify a detected fibre as a fibrillated fibre in response to fibrils connected to the fibre reaching a predetermined threshold parameter.

3.  The method of any preceding claim, wherein the method comprises:

    - recognizing an object in the at least one image,
    - classifying the object as a fibre or a fibril on the basis of one or more of determined length of the object, width of the object, and area of the object, and

    wherein the object is classified as the fibre, the method further comprises:

    - classifying the fibre as a non-fibrillated fibre or a fibrillated fibre on the basis of a determined area or a determined circle of the fibre.

4.  The method of any preceding claim, wherein the controlling fibre refining comprises controlling of further processing of pulp batch being analysed and refined into a further or a recurrent refining stage or to a subsequent process stage after the pulp refining.

5.  The method of any preceding claim, wherein the controlling fibre refining comprises controlling chemical treatment for chemical fibrillation of the fibrous pulp on the basis of the control parameter.

6. The method of any preceding claim, wherein internal fibrillation is analysed on the basis of the relation between fibrillated fibres and all fibres or non-fibrillated fibres in the pulp and/or by energy used in the refining of the pulp, wherein a fibre may be classified as an internally fibrillated fibre if a width of the fibre reaches predetermined threshold value.

7. An apparatus for monitoring and controlling of refining of fibrous pulp comprising:

   - an imaging module (304) configured for capturing at least one image of the pulp sample;
   - a fibre analysis module (308) configured for determining amount of all fibres or non-fibrillated fibres in the at least one image, **characterized in that** the apparatus comprises:
   - the fibre analysis module (308) configured for determining amount of fibrillated fibres in the at least one image, and
   determining relation between fibrillated fibres and all fibres or non-fibrillated fibres in the pulp on the basis of the amount of fibrillated fibres and the amount of all fibres or non-fibrillated fibres in the at least one image;
   - a process controller (318) configured for generating a control parameter on the basis of the determined relation between fibrillated fibres and all fibres or non-fibrillated fibres in the pulp; and
   - a mechanical refining controller (322) configured for causing control of fibre modification by at least one pulp refining device on the basis of the control parameter.

8. The apparatus of any preceding claim, wherein the means are configured to determine the amount of the fibrillated and/or all fibres by a fibre classifier (314) configured to:

   - detect fibres and fibrils of fibres in the at least one image, and
   - classify the detected fibre as a fibrillated fibre in response to fibrils connected to the fibre reaching a predetermined threshold parameter.

9. The apparatus of any preceding claim, wherein the means are configured to classify an object recognized in the at least one image as a fibre or a fibril on the basis of one or more of determined length of the object, width of the object, and area of the object.

10. The apparatus of any preceding claim, wherein the means are configured to classify a fibre recognized in the at least one image into the non-fibrillated fibre or the fibrillated fibre on the basis of a determined area or a determined circle of the fibre.

11. The apparatus of any preceding claim, wherein the controlling fibre refining comprises controlling of further processing of pulp batch being analysed and refined into a further or a recurrent refining stage or to a subsequent process stage after the pulp refining.

12. The apparatus of any preceding claim, wherein the controlling fibre refining comprises controlling chemical treatment for chemical fibrillation of the fibrous pulp on the basis of the control parameter.

13. The apparatus of any preceding claim, which the means are configured to analyse internal fibrillation on the basis of the relation between fibrillated fibres and all fibres or non-fibrillated fibres in the pulp and/or by energy used in the refining of the pulp wherein a fibre may be classified as an internally fibrillated fibre if a width of the fibre reaches predetermined threshold value.

14. The apparatus of any preceding claim 7 to 13, wherein the means are further configured for performing the method of any one of claims 1 to 6.

15. A fibrous pulp processing system, **characterized in that** the fibrous pulp processing system comprises the apparatus of any preceding claim 7 to 14.

**Patentansprüche**

1. Verfahren zur Überwachung und Steuerung der Mahlung von Faserzellstoff, wobei das Verfahren Folgendes umfasst:

- Aufnehmen (200) mindestens eines Bildes einer Zellstoffprobe durch ein Bildgebungsmodul (304);
- Bestimmen (202) einer Menge aller Fasern oder nicht-fibrillierter Fasern in dem mindestens einen Bild mittels eines Faseranalysemoduls (308); **gekennzeichnet durch**
- Bestimmen (204) einer Menge fibrillierter Fasern in dem mindestens einen Bild auf Grundlage der Bildverarbeitung durch das Faseranalysemodul (308);
- Bestimmen (206) eines Verhältnisses zwischen fibrillierten Fasern und allen Fasern oder nicht fibrillierten Fasern im Zellstoff auf der Grundlage der Menge an fibrillierten Fasern und der Menge an allen Fasern oder nicht fibrillierten Fasern in dem mindestens einen Bild durch das Faseranalysemodul (308);
- Erzeugen (208) eines Steuerparameters auf der Grundlage des bestimmten Verhältnisses zwischen fibrillierten Fasern und allen Fasern oder nicht fibrillierten Fasern im Zellstoff durch eine Prozesssteuereinheit (318), und
- Steuern (210) einer Fasermahlung mittels mindestens einer Zellstoffmahlvorrichtung auf Basis des Steuerparameters durch die mechanische Mahlungssteuereinheit (322).

2. Verfahren nach einem vorstehenden Anspruch, wobei die Menge der fibrillierten und aller Fasern durch einen Faserklassifikator (314) bestimmt wird, der konfiguriert ist:

- Fasern und Faserfibrillen in dem mindestens einen Bild zu erkennen, und
- eine erkannte Faser als fibrillierte Faser zu klassifizieren, wenn die mit der Faser verbundenen Fibrillen einen vorbestimmten Schwellenwert erreichen.

3. Verfahren nach einem vorstehenden Anspruch, wobei das Verfahren umfasst:

- Erkennen eines Objekts in dem mindestens einen Bild,
- Klassifizieren des Objekts als Faser oder Fibrille auf der Grundlage eines oder mehrerer von bestimmter Länge, Breite und/oder Fläche des Objekts und wobei das Objekt als Faser klassifiziert wird, wobei das Verfahren ferner umfasst:
- Klassifizieren der Faser als nicht fibrillierte Faser oder fibrillierte Faser auf der Grundlage einer bestimmten Fläche oder eines bestimmten Kreises der Faser.

4. Verfahren nach einem vorstehenden Anspruch, wobei das Steuern der Fasermahlung Steuern einer Weiterverarbeitung der analysierten und gemahlenen Zellstoffcharge in eine weitere oder wiederkehrende Mahlungsstufe oder in eine nachfolgende Prozessstufe nach der Zellstoffmahlung umfasst.

5. Verfahren nach einem vorstehenden Anspruch, wobei das Steuern der Fasermahlung Steuern einer chemischen Behandlung zur chemischen Fibrillierung des Faserzellstoffs auf der Grundlage des Steuerparameters umfasst.

6. Verfahren nach einem vorstehenden Anspruch, wobei innere Fibrillation auf der Grundlage des Verhältnisses zwischen fibrillierten Fasern und allen Fasern oder nicht fibrillierten Fasern im Zellstoff und/oder durch die bei der Mahlung des Zellstoffs aufgewendete Energie analysiert wird,
wobei eine Faser als intern fibrillierte Faser klassifiziert werden kann, wenn eine Breite der Faser einen vorbestimmten Schwellenwert erreicht.

7. Einrichtung zur Überwachung und Steuerung der Mahlung von Faserzellstoff, umfassend:

- ein Bildgebungsmodul (304), das so konfiguriert ist, dass es mindestens ein Bild der Zellstoffprobe aufnimmt;
- ein Faseranalysemodul (308) das so konfiguriert ist, dass es eine Menge aller Fasern oder nicht fibrillierter Fasern in dem mindestens einen Bild bestimmt, **dadurch gekennzeichnet, dass** die Einrichtung Folgendes umfasst:

  - das Faseranalysemodul (308), das so konfiguriert ist, dass es die Menge fibrillierter Fasern in dem mindestens einen Bild bestimmt und
  ein Verhältnis zwischen fibrillierten Fasern und allen Fasern oder nicht fibrillierten Fasern im Zellstoff auf der Grundlage der Menge an fibrillierten Fasern und der Menge an allen Fasern oder nicht fibrillierten Fasern in dem mindestens einen Bild bestimmt;
  - eine Prozesssteuereinheit (318), die so konfiguriert ist, dass sie einen Steuerparameter auf der Grundlage des bestimmten Verhältnisses zwischen fibrillierten Fasern und allen Fasern oder nicht fibrillierten Fasern im Zellstoff erzeugt; und
  - eine mechanische Mahlsteuereinheit (322), die so konfiguriert ist, dass sie eine Steuerung einer Fasermodifikation durch mindestens eine Zellstoffmahlvorrichtung auf der Grundlage des Steuerparameters veranlasst.

8. Einrichtung nach einem vorstehenden Anspruch, wobei die Mittel so konfiguriert sind, dass sie die Menge der fibrillierten und/oder aller Fasern durch einen Faserklassifikator (314) bestimmen, der konfiguriert ist:

- Fasern und Faserfibrillen in dem mindestens einen Bild zu erkennen, und
- die erkannte Faser als fibrillierte Faser zu klassifizieren, wenn die mit der Faser verbundenen Fibrillen einen vorbestimmten Schwellenwert erreichen.

9. Einrichtung nach einem vorstehenden Anspruch, wobei die Mittel so konfiguriert sind, dass sie ein in dem mindestens einen Bild erkanntes Objekt als Faser oder Fibrille auf der Grundlage eines oder mehrerer von bestimmter Länge des Objekts, Breite des Objekts und Fläche des Objekts klassifizieren.

10. Einrichtung nach einem vorstehenden Anspruch, wobei die Mittel so konfiguriert sind, dass sie eine in dem mindestens einen Bild erkannte Faser auf Grundlage einer bestimmten Fläche oder eines bestimmten Kreises der Faser in nicht-fibrillierte Faser oder fibrillierte Faser klassifizieren.

11. Einrichtung nach einem vorstehenden Anspruch, wobei das Steuern der Fasermahlung Steuern einer Weiterverarbeitung der analysierten und gemahlenen Zellstoffcharge in eine weitere oder wiederkehrende Mahlungsstufe oder in eine nachfolgende Prozessstufe nach der Zellstoffmahlung umfasst.

12. Einrichtung nach einem vorstehenden Anspruch, wobei das Steuern der Fasermahlung Steuern einer chemischen Behandlung zur chemischen Fibrillierung des Faserzellstoffs auf der Grundlage des Steuerparameters umfasst.

13. Einrichtung nach einem vorstehenden Anspruch, deren Mittel so konfiguriert sind, dass sie die interne Fibrillation auf der Grundlage des Verhältnisses zwischen fibrillierten Fasern und allen Fasern oder nicht fibrillierten Fasern im Zellstoff und/oder durch die bei der Mahlung des Zellstoffs aufgewendete Energie analysieren, wobei eine Faser als intern fibrillierte Faser klassifiziert werden kann, wenn eine Breite der Faser einen vorbestimmten Schwellenwert erreicht.

14. Einrichtung nach einem der vorstehenden Ansprüche 7 bis 13, wobei die Mittel ferner zum Durchführen des Verfahrens nach einem der Ansprüche 1 bis 6 konfiguriert sind.

15. Faserzellstoffverarbeitungssystem, **dadurch gekennzeichnet, dass** das Faserzellstoffverarbeitungssystem die Einrichtung nach einem vorstehenden Anspruch 7 bis 14 umfasst.

## Revendications

1. Procédé de surveillance et de commande du raffinage d'une pâte fibreuse, le procédé comprenant :

- la capture (200) d'au moins une image d'un échantillon de pâte par un module d'imagerie (304) ;
- la détermination (202) d'une quantité de toutes les fibres ou fibres non fibrillées dans la au moins une image par un module d'analyse de fibres (308) ; **caractérisé par**
- la détermination (204) d'une quantité de fibres fibrillées dans la au moins une image sur la base d'un traitement d'image par le module d'analyse de fibres (308) ;
- la détermination (206) d'une relation entre des fibres fibrillées et toutes les fibres ou fibres non fibrillées dans la pâte sur la base de la quantité de fibres fibrillées et de la quantité de toutes les fibres ou fibres non fibrillées dans la au moins une image par le module d'analyse de fibres (308) ;
- la génération (208) d'un paramètre de commande sur la base de la relation déterminée entre des fibres fibrillées et toutes les fibres ou fibres non fibrillées dans la pâte par un dispositif de commande de processus (318), et
- la commande (210) du raffinage de fibres par au moins un dispositif de raffinage de pâte sur la base du paramètre de commande par un dispositif de commande de raffinage mécanique (322).

2. Procédé selon une quelconque revendication précédente, dans lequel la quantité des fibres fibrillées et de toutes les fibres est déterminée par un classifieur de fibres (314) configuré pour :

- détecter des fibres et des fibrilles de fibres dans la au moins une image, et
- classer une fibre détectée comme une fibre fibrillée en réponse à des fibrilles reliées à la fibre atteignant un paramètre de seuil prédéterminé.

3. Procédé selon une quelconque revendication précédente, dans lequel le procédé comprend :

- la reconnaissance d'un objet dans la au moins une image,
- le classement de l'objet comme une fibre ou une fibrille sur la base d'une ou plusieurs parmi la longueur déterminée de l'objet, la largeur déterminée de l'objet et la surface déterminée de l'objet, et dans lequel l'objet est classé comme la fibre, le procédé comprend en outre :

- le classement de la fibre comme une fibre non fibrillée ou une fibre fibrillée sur la base d'une surface déterminée ou d'un cercle déterminé de la fibre.

4. Procédé selon une quelconque revendication précédente, dans lequel la commande de raffinage de fibres comprend la commande d'un traitement supplémentaire d'un lot de pâte en cours d'analyse et de raffinage lors d'une étape de raffinage supplémentaire ou récurrente ou jusqu'à une étape de traitement supplémentaire après le raffinage de pâte.

5. Procédé selon une quelconque revendication précédente, dans lequel la commande de raffinage de fibres comprend la commande d'un traitement chimique pour la fibrillation chimique de la pâte fibreuse sur la base du paramètre de commande.

6. Procédé selon une quelconque revendication précédente, dans lequel une fibrillation interne est analysée sur la base de la relation entre des fibres fibrillées et toutes les fibres ou fibres non fibrillées dans la pâte et/ou par l'énergie utilisée dans le raffinage de la pâte,
dans lequel une fibre peut être classée comme une fibre fibrillée intérieurement si une largeur de la fibre atteint une valeur de seuil prédéterminée.

7. Appareil de surveillance et de commande du raffinage d'une pâte fibreuse comprenant :

- un module d'imagerie (304) configuré pour capturer au moins une image de l'échantillon de pâte ;
- un module d'analyse de fibres (308) configuré pour déterminer une quantité de toutes les fibres ou fibres non fibrillées dans la au moins une image, **caractérisé en ce que** l'appareil comprend :

- le module d'analyse de fibres (308) configuré pour déterminer une quantité de fibres fibrillées dans la au moins une image, et
déterminer une relation entre des fibres fibrillées et toutes les fibres ou fibres non fibrillées dans la pâte sur la base de la quantité de fibres fibrillées et de la quantité de toutes les fibres ou fibres non fibrillées dans la au moins une image ;
- un dispositif de commande de processus (318) configuré pour générer un paramètre de commande sur la base de la relation déterminée entre des fibres fibrillées et toutes les fibres ou fibres non fibrillées dans la pâte ; et
- un dispositif de commande de raffinage mécanique (322) configuré pour provoquer la commande d'une modification de fibres par au moins un dispositif de raffinage de pâte sur la base du paramètre de commande.

8. Appareil selon une quelconque revendication précédente, dans lequel les moyens sont configurés pour déterminer la quantité des fibres fibrillées et/ou de toutes les fibres grâce à un classifieur de fibres (314) configuré pour :

- détecter des fibres et des fibrilles de fibres dans la au moins une image, et
- classer la fibre détectée comme une fibre fibrillée en réponse à des fibrilles reliées à la fibre atteignant un paramètre de seuil prédéterminé.

9. Appareil selon une quelconque revendication précédente, dans lequel les moyens sont configurés pour classer un objet reconnu dans la au moins une image comme une fibre ou une fibrille sur la base d'une ou plusieurs parmi la longueur déterminée de l'objet, la largeur déterminée de l'objet et la surface déterminée de l'objet.

10. Appareil selon une quelconque revendication précédente, dans lequel les moyens sont configurés pour classer une fibre reconnue dans la au moins une image en la fibre non fibrillée ou la fibre fibrillée sur la base d'une surface déterminée ou d'un cercle déterminé de la fibre.

11. Appareil selon une quelconque revendication précédente, dans lequel la commande de raffinage de fibres comprend la commande d'un traitement supplémentaire d'un lot de pâte en cours d'analyse et de raffinage lors d'une étape de raffinage supplémentaire ou récurrente ou jusqu'à une étape de processus supplémentaire après le raffinage de pâte.

12. Appareil selon une quelconque revendication précédente, dans lequel la commande de raffinage de fibres comprend la commande d'un traitement chimique pour la fibrillation chimique de la pâte fibreuse sur la base du paramètre de commande.

13. Appareil selon une quelconque revendication précédente, dans lequel les moyens sont configurés pour analyser la fibrillation interne sur la base de la relation entre des fibres fibrillées et toutes les fibres ou fibres non fibrillées dans la pâte et/ou par l'énergie utilisée dans le raffinage de la pâte, dans lequel une fibre peut être classée comme une fibre fibrillée intérieurement si une largeur de la fibre atteint une valeur de seuil prédéterminée.

14. Appareil selon l'une quelconque des revendications précédentes 7 à 13, dans lequel les moyens sont en outre configurés pour exécuter le procédé selon l'une quelconque des revendications 1 à 6.

15. Système de traitement de pâte fibreuse, **caractérisé en ce que** le système de traitement de pâte fibreuse comprend l'appareil selon l'une quelconque des revendications précédentes 7 à 14.

Fig. 1

CAPTURING IMAGE — 200

DETERMINING AMOUNT OF ALL FIBRES OR NON-FIBRILLATED FIBRES — 202

DETERMINING AMOUNT OF FIBRILLATED FIBRES — 204

DETERMINING RELATION BETWEEN FIBRILLATED FIBRES AND ALL FIBRES OR NON-FIBRILLATED FIBRES — 206

GENERATING CONTROL PARAMETER — 208

CONTROLLING FIBRE REFINING — 210

Fig. 2

Fig. 3

Fig. 4

Fig. 5

Fig. 6

**EP 3 733 959 B1**

**REFERENCES CITED IN THE DESCRIPTION**

**Patent documents cited in the description**

- JP 2013186034 A

- WO 9915877 A